# EUROPEAN PATENT SPECIFICATION

(11) **EP 2 769 298 B1**
(45) Date of publication and mention of the grant of the patent: **13.12.2017**
(21) Application number: 12784396.9
(22) Date of filing: 19.10.2012
(51) Int. Cl.: G06F 9/445, G06F 9/50

(54) **SECURE AUTOMATIC CONFIGURATION OF EQUIPMENT THROUGH REPLICATION**
SICHERE AUTOMATISCHE KONFIGURIERUNG EINER VORRICHTUNG MITTELS REPLIKATION
CONFIGURATION AUTOMATIQUE SÉCURISÉE D'ÉQUIPEMENT PAR UNE REPRODUCTION

(30) Priority: 19.10.2011 US 201161549130 P; 19.10.2011 US 201161549202 P; 18.10.2012 US 201213655369
(43) Date of publication of application: 27.08.2014
(73) Proprietor: Qualcomm Incorporated, San Diego, CA 92121 (US)
(72) Inventor: MARSH, Gene Wesley, San Diego California 92121 (US); ANAND, Rahul, San Diego California 92121 (US); VAUGHN, Brad L., San Diego California 92121 (US); CHEN, Jen Mei, San Diego California 92121 (US); CHEN, Tao, San Diego California 92121 (US); GUO, Jin, San Diego California 92121 (US); SINGH, Navrina B., San Diego California 92121 (US); DOROBA, Joseph, San Diego California 92121 (US); ZHANG, Jiang, San Diego California 92121 (US)
(74) Representative: Dunlop, Hugh Christopher
(86) International application number: PCT/US2012/061176
(87) International publication number: WO 2013/059709

(56) References cited:
- US-A1- 2005 210 525
- US-A1- 2007 038 823
- US-A1- 2007 190 939
- US-A1- 2008 228 832
- US-B1- 6 636 873
- Dejan Milojicic ET AL: "Process Migration", , 1 February 1999 (1999-02-01), pages 0-48, XP055012410, Palo Alto Retrieved from the Internet: URL:http://www.hpl.hp.com/techreports/1999 /HPL-1999-21.pdf [retrieved on 2011-11-17]

## Description

### BACKGROUND

### Field

Certain aspects of the present disclosure generally relate to wireless communications and, more particularly, to techniques for secure automatic configuration of equipment through replication.

### Background

As various computer, electrical, and mechanical techniques have evolved, many different types of equipment have achieved increased scope and functionality to perform many new and increasingly complicated tasks. With this increased complexity, the control and configuration of such equipment are also becoming more complicated, time consuming, and potentially more error prone.

An example of background art in the field is Cho et al., "Optimal beacon scheduling mechanisms using cluster identifier for healthcare application", Expert Systems with Applications, pp. 5071-5080, Vol. 36, Issue 3, 2009.

US 2005/210525 (Carle - Kevin) concerns a method of initializing a client device that has an associated identifier. The identifier is communicated to a configuration server that contains configuration information associated with the client device. The configuration information is received from the configuration server and is applied to the client device.

US 2007/190939 (Abel Miller) concerns a device provisioning and/or configuration system and method, wherein the system employs a near field communication (NFC) channel in an active and/or passive mode as a channel for provisioning and/or managing mobile device(s) (e.g., with content and/or setting(s)) by a server.

### SUMMARY

Aspects of the present invention are defined by the accompanying claims. In accordance with a first aspect there is provided a method of replicating configuration information at a first medical device in a healthcare environment in accordance with claim 1. In accordance with a second aspect there is provided a medical apparatus for replicating configuration information in accordance with claim 9. In accordance with a third aspect there is provided a first medical device in accordance with claim 13..

### BRIEF DESCRIPTION OF THE DRAWINGS

So that the manner in which the above-recited features of the present disclosure can be understood in detail, a more particular description, briefly summarized above, may be had by reference to aspects, some of which are illustrated in the appended drawings. It is to be noted, however, that the appended drawings illustrate only certain typical aspects of this disclosure and are therefore not to be considered limiting of its scope, for the description may admit to other equally effective aspects.
FIG. 1 illustrates a diagram of an example communications network in accordance with certain aspects of the present disclosure.
FIG. 2 illustrates a block diagram of an example wireless device in accordance with certain aspects of the present disclosure.
FIG. 3 illustrates an example system for transferring configuration information from a source device to a target device in accordance with certain aspects of the present disclosure.
FIG. 4 illustrates an example operation for replicating configuration information in accordance with certain aspects of the present disclosure.
FIG. 4A illustrates an example means for performing the operations shown in FIG. 4.

### DETAILED DESCRIPTION

Various aspects of the disclosure are described more fully hereinafter with reference to the accompanying drawings.

### AN EXAMPLE COMMUNICATION SYSTEM

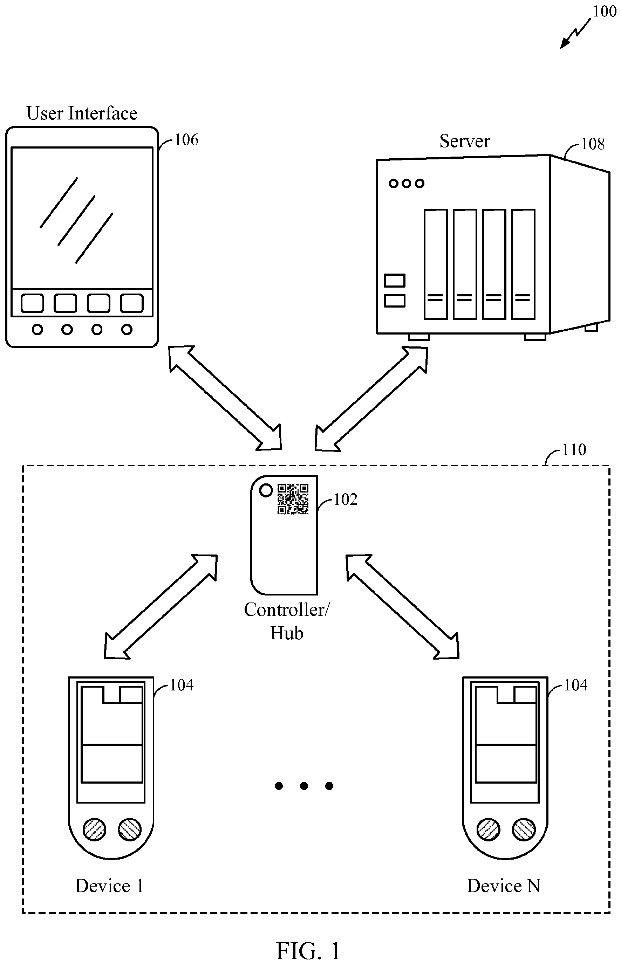

FIG. 1 illustrates an example distributed communication system 100 with a controller/hub 102 and one or more wireless devices 104. The controller/hub 102 may communicate with the wireless devices 104 via one or more wireless channels using one or more antennas and/or via one or more wired connections. The controller/hub 102 may function similar to an access point (AP) or a Wi-Fi hotspot in an IEEE 802.11 network.

The communication system 100 may be used in a healthcare environment, such as a hospital, clinic, hospice, or home. In such cases, the wireless devices 104 may include any of various suitable wireless medical devices, such as an infusion pump, a blood pressure monitor, a pulse oximeter, an electrocardiograph (ECG), and the like. The controller/hub 102 may be associated with a single patient, and the controller/hub 102 and the wireless devices associated therewith may form an in-room network 110. The in-room network 110 may function similar to a local area network (LAN) or a home network. Certain wireless devices 104 may be worn by the patient (e.g., a finger-worn or wrist-worn unit); inserted or implanted into the patient's body; or attached to or embedded in the patient's bed, gurney, clothing, or other devices that would generally stay physically close to the patient (e.g., a walker, cane, watch, or glasses).

Wireless communication between the controller/hub 102 and a wireless device 104 may use any of various suitable wireless technologies, such as near field communication (NFC), Bluetooth, Bluetooth Low Energy (BTLE), Wi-Fi in accordance with the IEEE 802.11 standard, Zigbee, ANT/ANT+, Toumaz Healthcare Ltd.'s Sensium™ platform for developing body area networks (BANs), Medical Implant Communication Service (MICS), and the like. The wireless communication may also occur via infrared (IR), bar code scanning, or other optical technologies.

A user interface 106 may provide an interface for a user (e.g., a doctor, a nurse, or the patient himself) to communicate with the controller/hub 102. The user interface 106 may comprise a networked device, such as a tablet (as shown), a smart phone, a cellular phone, a laptop, or dedicated electronic hardware. The user interface 106 may be connected with the controller/hub 102, a healthcare facility intranet (e.g., a hospital intranet), or the wireless device 104 directly via physical wires, wirelessly, or both. The connection between the user interface 106 and the controller/hub 102 or the wireless device 104 may be part of the in-room network 110.

A server 108 may provide an interface between the controller/hub 102 and a healthcare facility intranet. As part of the healthcare information system (HIS), the server 108 may store and provide access to electronic medical records (EMRs) of the patients and may provide the intelligence for checking therapies against patient allergies, preventing conflicting medications, etc. For certain aspects, the controller/hub 102 may provide the only interface between the in-room network 110 and the facility intranet. The controller/hub 102 may be connected with the facility intranet directly via a wired technology (e.g., Ethernet), indirectly via a wireless router connected to the server 108 via a wired technology, or indirectly via a public or private wired, wireless, or hybrid network technology.

One example scenario of the interactions between the various apparatus in the communication system 100 in a healthcare environment involves drug delivery to a patient. First, an infusion pump (e.g., a wireless device 104) may scan the patient's identification (ID), which may be contained in a bar code wristband worn by the patient. Then NFC may be used for out-of-band pairing between the controller/hub 102 and the pump. The pump may inform the controller/hub 102 of the patient's ID. Based on this, the controller/hub 102 may query the HIS/EMR for the patient's treatment information. Once this information is received, the controller/hub 102 may configure the pump (i.e., may transmit configuration information to the pump) for a particular intravenous (IV) therapy treatment. The configuration information may include the set of solution and medication, the solution's density, the flow rate, the total volume to infuse, and an interval (in an intermittent flow pattern) for an intravenous therapy the patient is prescribed to receive. The user interface 106 may prompt the caregiver to confirm the treatment, and once the caregiver confirms, the caregiver may scan a bar code, a quick response (QR) code, or a radio frequency identification (RFID), for example, on an IV solution bag before or after the caregiver connects the bag with the pump. The controller/hub 102 may signal the infusion pump to begin the treatment, perhaps at the command of the caregiver. Data from the pump may be transmitted to the controller/hub 102, and particular received data may be transmitted to the HIS via the healthcare facility intranet or other wireless or wired links.

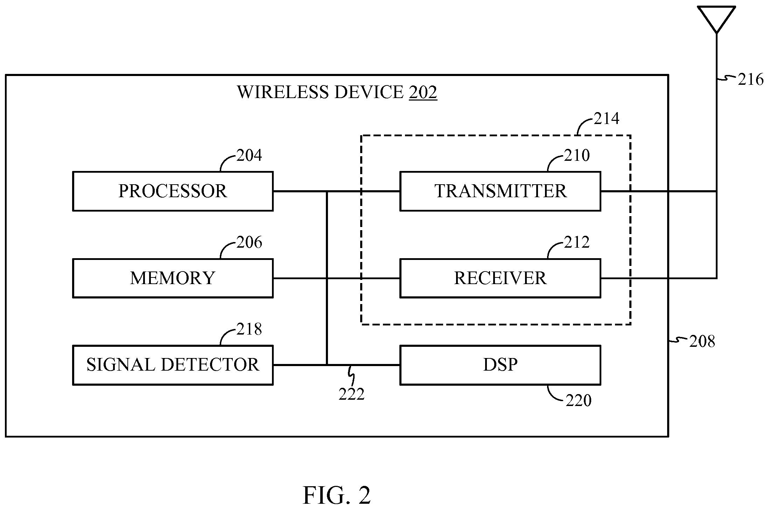

FIG. 2 illustrates various components that may be utilized in a wireless device 202. The wireless device 202 is an example of a device that may be configured to implement the various methods described herein. The wireless device 202 may be a controller/hub 102 or a wireless device 104, as described above with respect to FIG. 1.

The wireless device 202 may include a processor 204 which controls operation of the wireless device 202. The processor 204 may also be referred to as a central processing unit (CPU). Memory 206, which may include both read-only memory (ROM) and random access memory (RAM), provides instructions and data to the processor 204. A portion of the memory 206 may also include non-volatile random access memory (NVRAM). The processor 204 typically performs logical and arithmetic operations based on program instructions stored within the memory 206. The instructions in the memory 206 may be executable to implement the methods described herein.

The wireless device 202 may also include a housing 208 that may include a transmitter 210 and a receiver 212 to allow transmission and reception of data between the wireless device 202 and a remote location. The transmitter 210 and receiver 212 may be combined into a transceiver 214. An antenna 216 may be attached to the housing 208 and electrically coupled to the transceiver 214. The wireless device 202 may also include (not shown) multiple transmitters, multiple receivers, multiple transceivers, and/or multiple antennas.

The wireless device 202 may also include a signal detector 218 that may be used in an effort to detect and quantify the level of signals received by the transceiver 214. The signal detector 218 may detect such signals as total energy, pilot energy from pilot subcarriers or signal energy from the preamble symbol, power spectral density, and other signals. The wireless device 202 may also include a digital signal processor (DSP) 220 for use in processing signals.

The various components of the wireless device 202 may be coupled together by a bus system 222, which may include a power bus, a control signal bus, and a status signal bus in addition to a data bus.

### EXAMPLE SECURE AUTOMATIC CONFIGURATION OF EQUIPMENT THROUGH REPLICATION

Medical equipment is becoming increasingly computerized. While this provides many benefits, it also frequently involves cumbersome configuration procedures. In many cases, a new piece of medical equipment, for example, may be replacing another that has insufficient battery charge, is no longer considered sterile, has sustained damages, etc. Typically, when a new piece of equipment replaces an already configured piece of equipment, the new equipment is configured in the same manner as the original equipment was configured. The configuration process may most likely be repeated for every change of the equipment. For instance, a patient may be continually given a particular intravenous (IV) infusion via a pump. As one round of treatment ends, another bag is brought in, and the complete configuration of the original treatment is normally repeated.

In certain aspects, if the two pieces of equipment possess the capability to communicate with each other, it is possible to pass on configuration information from the old equipment to the new equipment, thereby easing the configuration burden. In certain aspects, if a replicating device is able to communicate with the devices whose functionality it is replicating, directly or via a third (wired or wireless) device, then a protocol may be established whereby the replacing device may receive the configuration of the device it is replacing, suitably verified and optionally modified. For certain aspects, the third device may be a controller of the two devices. This replication considerably reduces the amount of configuration data entry inputted by a caregiver and may most likely result in a reduction of errors in those configurations. Also, in an emergency, such as an epidemic outbreak of a contagious disease or after a natural disaster, a common medical treatment may easily be configured for many people at once. Furthermore, all devices in a system may be handed over from one controller to another using this configuration capability.

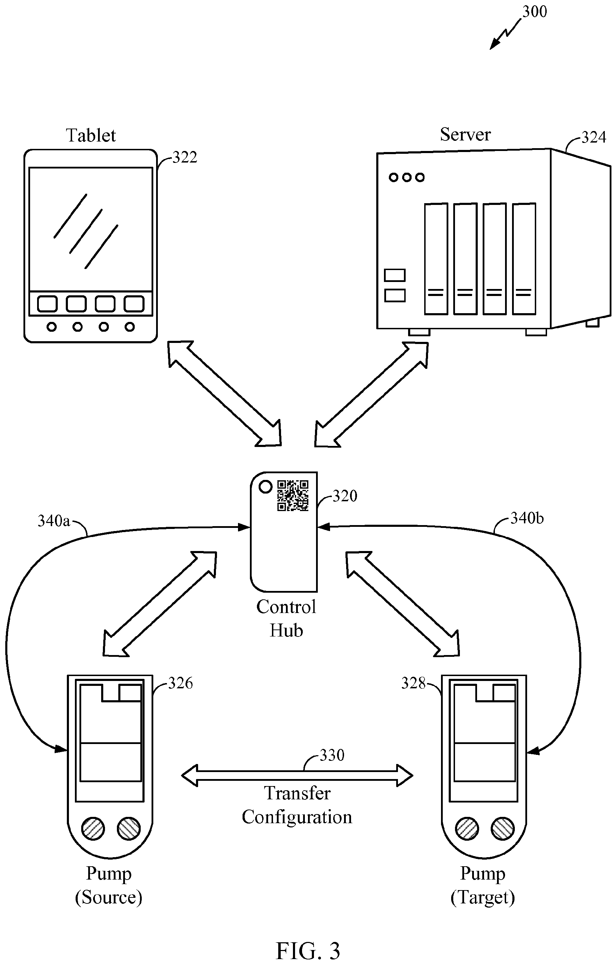

FIG. 3 illustrates an example system 300 for transferring configuration information from a source device (such as an infusion pump) to a target device in accordance with certain aspects of the present disclosure. The system 300 includes a tablet 322, a server 324, a source pump 326, and a target pump 328 communicatively coupled to a control hub 320 (e.g., controller/hub 102 shown in FIG. 1).

In certain aspects, the source and target pumps 326, 328 and the control hub 320 are part of an in-room network 110 typically associated with a single patient. The control hub 320 typically interfaces with the tablet 322 and the server 324 and controls all other in-room equipment, such as the pumps 326, 328 a blood pressure monitor, a pulse oximeter, an electrocardiograph (ECG), or a patient's wrist-worn unit. The control hub 320 may control the in-room equipment through updating the configuration information or setting or adjusting one or more particular operational parameters. The control hub 320 may be communicatively coupled to the other in-room equipment, the tablet 322, and the server 324 via wired or wireless links and may provide access to the other in-room equipment through a user interface device, such as the tablet 322.

The server 324 typically provides an interface between the control hub 320 and a healthcare network (e.g., a hospital network) (not shown); provides access to information such as work lists, patient records, information about medicines to be delivered, etc.; and relays status reports from the control hub 320. The tablet 322 provides a user interface for the system 300. The tablet 322 may be used by a caregiver (e.g., a nurse, physician's assistant (PA), doctor, etc. in a hospital or clinic, for example) to access information on the server 324 and the healthcare network via the server 324. The tablet 322 may also be used by the caregiver to issue commands to the control hub 320 and the medical equipment via the control hub 320, and to view status of the network and the equipment. Pumps 326, 328 are used for medication delivery and typically control a rate of flow of medication to a patient. A pump 326, 328 may be associated with a particular intravenous (IV) bag, for example, identified by a unique bar code which may be scanned by the pump. The in-room equipment may further include patient-worn sensors such as an electrocardiograph (ECG), a blood pressure monitoring device, a pulse oximeter, a thermometer, and the like.

In certain aspects, the system enables transferring configuration information between devices of the in-room network 110 directly or via the control hub. For example, a caregiver wishing to duplicate the programming of a source pump 326 in a target pump 328 without using the control hub 320 may begin by optionally authenticating himself/herself to each of the pumps. This authentication may be accomplished, for example, using an electronic credential such as a user device equipped with a certificate, or a user name and password pair, etc. The caregiver may then associate the target pump 328 with the source pump 326 using a secure communication channel 330. The source pump 326 may then autonomously or at the caregiver's command transfer its configuration data to the target pump 328 via the communication channel 330.

In certain aspects, the configuration data may be encrypted and/or protected with a secure checksum in an effort to ensure integrity of the data. The configuration data may, for example, include parameters desired for control of the source pump 326, a current status of its operation, data logged for record keeping or performance tracking, progress of a treatment administered by the pump, etc. In an aspect, the caregiver may be given an opportunity to modify the configuration data at the source pump 326 before the transfer or at the target pump 328 after the transfer. The caregiver may then decommission the source pump 326 and commence the operation of the target pump 328 (in either order or simultaneously), possibly continuing the operation from where the source pump 326 left off. In this configuration, the source and/or the target pumps may be virtual devices, as represented, for example, in the database of a device controller.

In certain aspects, a caregiver wishing to duplicate the programming of the source pump via the control hub 320 would begin by authenticating himself/herself to the control hub 320. This authentication may be accomplished using an electronic credential such as a user device equipped with a certificate, or a user name and password pair, or the like. The caregiver may then associate the target pump 328 with the control hub 320. After selecting the source pump 326, the control hub 320 may be commanded to perform the replication. The control hub 320 may fetch the configuration of the source pump 326, either by querying it over an encrypted channel 340a, or from a cache, and then present that configuration to the caregiver (e.g., on the tablet 322). The configuration may, for example, include parameters desired for control of the source pump 326, the current status of its operation, data logged for performance tracking, progress of a treatment, etc. The caregiver may be given an opportunity to modify the configuration data using the tablet 322, for example, before commanding the control hub 320 to transfer the configuration data. The caregiver may then select the target pump 328 for the transfer. The control hub 320 may establish a secure link 340b to the target pump 328 and then transfer the configuration information to the target pump 328 via the link 340b. In an aspect, once the target pump 328 is successfully configured, the source pump 326 may be decommissioned, and operation of the target pump 328 may then begin, possibly continuing the operation from where the source device left off.

In certain aspects, the caregiver may select more than one target device for the transfer, which may be already associated devices or newly associated devices. In an aspect, a secure broadcast or multicast link may be established to multiple target devices, and the configuration data may be transferred in parallel to these target devices.

The mechanisms described above may be combined to enable other applications. For instance, one may propagate a particular configuration across an extended network of devices. As another example, one may manage the transfer of devices between two controllers. In this latter example, one of the two controllers may be active and has a number of devices associated with it, and the other of the two controllers has not yet been configured and has no devices. The device currently designated as the controller may be referred to as the source, and the other controller may be designated as the target. Transfer of configuration data using the two-device implementation above may likely provide all of the data specified for the target controller to assume the duties of the source controller. An additional operation may be initiated that transfers the secure link information for the target controller into the devices managed by the source controller, resulting in a smooth transfer of control. In another aspect, each device managed by the source controller may individually be re-associated with the target controller, such action triggering a transfer in the two-device implementation between the new device and the target controller. Once all devices are paired, the source controller may be safely decommissioned.

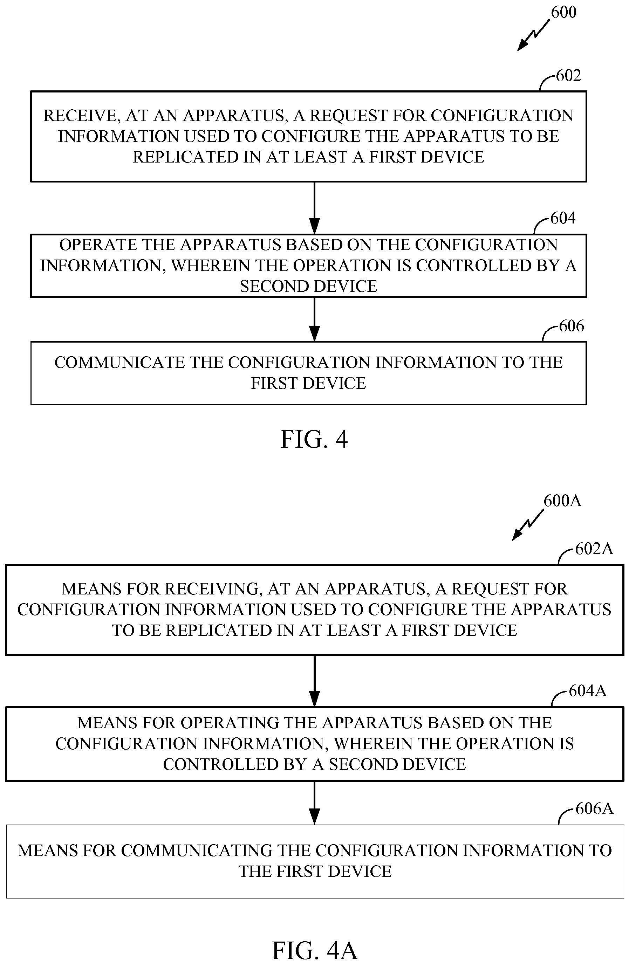

FIG. 4 illustrates example operations 600 for replicating configuration information in accordance with certain aspects of the present disclosure. The operations 600 may be performed by an apparatus, such as a wireless device 104. The operations 600 may start, at 602, with the apparatus receiving a request for configuration information used to configure the apparatus to be replicated in at least a first (wireless-capable) device. The configuration information may include patient treatment information. At 604, the apparatus may be operated based on the configuration information, and this operation may be controlled by a second (wireless-capable) device (e.g., a controller/hub 102). At 606, the apparatus may communicate the configuration information to the first device, in response to the request received at 602.

According to certain aspects, the apparatus may determine whether to continue or stop the operation of the apparatus after receiving the request at 602. For certain aspects, the apparatus may cease the operation of the apparatus after the configuration information had been communicated at 606. For certain aspects, at least one of starting of the operation, stopping of the operation, monitoring progress of the operation, or managing an alarm based on the operation is controlled by the second device.

For certain aspects, the communicating at 606 includes transferring the configuration information to the first device via the second device. For certain aspects, the request is received from the second device, and the communicating comprises transmitting the configuration information to the second device, for transferring to the first device. The second device may provide an opportunity to adjust configuration information prior to transfer of the configuration information to the first device and, if the configuration information is adjusted, may communicate the adjusted configuration information to the first device. For certain aspects, the second device may control the apparatus and the first device. For certain aspects, the communicating may entail wirelessly transmitting the configuration information to the first device and one or more additional (wireless-capable) devices. The communicating may involve transferring the configuration information wirelessly for certain aspects.

According to certain aspects, the apparatus may adjust the configuration information after receiving the request at 602, but prior to communicating the configuration information to the first device at 606. For certain aspects, the configuration information is adjusted based on progress of a treatment administered thus far by the apparatus. For example, if a patient is to receive 1 L of a solution, and the apparatus has already administered 2/3 L, the configuration information may be adjusted to indicate only 1/3 L remains to be administered. For other aspects, the second device may adjust the configuration information after receipt. Such adjustment to a previously valid configuration may be made to perform the next phase of a treatment procedure, which may entail a different configuration than the previous phase.

According to certain aspects, each of the apparatus and the first device is a medical device, such as an infusion pump. In this case, the configuration information may include at least one of parameters for controlling the apparatus, a current status of operation of the apparatus, data logged in the apparatus for record keeping or performance tracking, or progress of a treatment administered by the apparatus which is optionally used for the next device (e.g., the first device) to continue a partially delivered treatment, or other cumulative records in the apparatus that may be appended to by the next device.

According to certain aspects, the apparatus may receive the request at 602 based on at least one of: a treatment provided by the apparatus has nearly or completely run out; the apparatus is no longer considered sterile; the apparatus has or nearly has insufficient battery charge; or the apparatus has sustained damage. For certain aspects, the apparatus may receive the request after the request is initiated or approved by another apparatus, such as a user interface 106.

According to certain aspects, the operations 600 may further include locally storing the configuration information in a memory of the apparatus. In this case, the apparatus may retrieve the configuration information from the memory in response to the request at 602.

For certain aspects, the request may be expressed by physically locating the apparatus and the first device close to each other such that a short range radio or a proximity sensor detects in one (or both of the devices can detect) the presence of the counterpart device (i.e., the other device). For certain aspects, the request is received if the apparatus is physically located close (e.g., within 1 m, or in some cases such as NFC, within 5 cm) to the first device.

The various operations of methods described above may be performed by any suitable means capable of performing the corresponding functions. The means may include various hardware and/or software component(s) and/or module(s), including, but not limited to a circuit, an application specific integrated circuit (ASIC), or processor. Generally, where there are operations illustrated in figures, those operations may have corresponding counterpart means-plus-function components with similar numbering. For example, operations 600 illustrated in FIG. 4 correspond to means 600A illustrated in FIG. 4A.

For example, means for transmitting or means for communicating may comprise a transmitter, such as the transmitter 210 of the wireless device 202 illustrated in FIG. 2. Means for receiving or means for communicating may comprise a receiver, such as the receiver 212 of the wireless device 202 shown in FIG. 2. Means for communicating, means for retrieving, means for obtaining, means for adjusting, means for operating, means for controlling, means for determining, and/or means for processing may comprise a processing system, which may include one or more processors, such as processor 204 illustrated in FIG. 2. Means for storing and/or means for retrieving may comprise a storage medium, such as memory 206 depicted in FIG. 2.

## Claims

1. A method for replicating configuration information at a first medical device (104, 328, 602) in a healthcare environment, **characterized by** comprising:
receiving, at a medical apparatus (104, 326) operating based on configuration information and controlled by a second device (102, 604), a request for the configuration information used to configure the medical apparatus (104, 326) to be replicated in the said first medical device (104, 328, 602);
wherein the configuration information comprises patient treatment information;
wherein the request is expressed by physically locating the first medical device close to the medical apparatus (104, 326), wherein physically located close means that a short range radio or a proximity sensor in one of the medical apparatus (104, 326) and the first medical device (104, 328, 602) detects the presence of the other one of the medical apparatus and the first medical device; and
communicating the configuration information to the first medical device, in response to the request (606).

2. The method of claim 1, further comprising ceasing the operation of the medical apparatus (104, 326) after the configuration information had been communicated.

3. The method of claim 1, wherein at least one of starting of the operation, stopping of the operation, monitoring progress of the operation, or managing an alarm based on the operation is controlled by the second device (102, 604).

4. The method of claim 1, wherein the communicating comprises transferring the configuration information to the first medical device (104, 328, 602) via the second device (102, 604).

5. The method of claim 1, further comprising adjusting the configuration information before communicating with the first medical device (104, 328, 602).

6. The method of claim 5, wherein the configuration information is adjusted based on progress of the operation of the medical apparatus (104, 326).

7. The method of claim 1, further comprising:
storing the configuration information in a memory at the medical apparatus (104, 326); and
retrieving the configuration information from the memory in response to the request.

8. The method of claim 1, wherein the first medical device is an infusion pump (104, 328, 602).

9. A medical apparatus for replicating configuration information in a healthcare environment, **characterized by** comprising:
a receiver configured to receive a request for configuration information used to configure the medical apparatus (104, 326) to be replicated in at least a first medical device (104, 328, 602) that is to replace the medical apparatus;
wherein the configuration information comprises patient treatment information;
short range radio means for facilitating receipt of the request if the medical apparatus is physically located close to the first medical device, wherein physically located close means that a short range radio or a proximity sensor in one of the medical apparatus (104, 326) and the first medical device (104, 328, 602) detects the presence of the other one of the medical apparatus and the first medical device;
a processing system configured to operate the medical apparatus based on the configuration information, wherein a second device is configured to control the operation; and
a transmitter configured to communicate the configuration information to the first medical device, in response to the request received by the receiver.

10. The medical apparatus of claim 9, wherein the medical apparatus is worn by a patient, or inserted or implanted into a patient's body, or attached to or embedded in a patient's bed, gurney, clothing.

11. The medical apparatus of claim 9 or 10, wherein the configuration information comprises at least one of:
parameters for controlling the apparatus;
a current status of operation of the apparatus;
data logged in the apparatus; or
progress of a treatment administered by the apparatus.

12. A computer-readable medium having stored thereon processor executable instructions that cause a computer to perform the method of any of claims 1 to 8 when executed by a computer.

13. A first medical device for receiving replicated configuration information in a healthcare environment, **characterized by** comprising:
a transmitter configured to communicate a request for configuration information used to configure a medical apparatus (104, 326) to be replicated in the first medical device (104, 328, 602);
wherein the configuration information comprises patient treatment information;
means for determining that the first medical device is physically located close to the medical apparatus and for expressing the request in response thereto, wherein physically located close means that a short range radio or a proximity sensor in one of the first medical device and the medical apparatus detects the presence of the other one of the first medical device and the medical apparatus;
a receiver configured to receive the configuration information; and
a processing system configured to operate the first medical device, based on the configuration information, under control of a second device.

## Patentansprüche

1. Ein Verfahren zum Replizieren von Konfigurationsinformationen an einem ersten medizinischen Gerät (104, 328, 602) in einer medizinischen Umgebung, **gekennzeichnet durch**:
Empfangen, an einer medizinischen Vorrichtung (104, 326), die basierend auf Konfigurationsinformationen betrieben wird und durch ein zweites Gerät (104, 604) gesteuert wird, einer Anfrage, dass die Konfigurationsinformationen, die für das Konfigurieren der medizinischen Vorrichtung (104, 326) verwendet werden, in dem ersten medizinischen Gerät (104, 328, 602) repliziert werden,
wobei die Konfigurationsinformationen Patientenbehandlungsinformationen aufweisen,
wobei die Anfrage gestellt wird, indem das erste medizinische Gerät physikalisch nahe an der medizinischen Vorrichtung (104, 326) positioniert wird, wobei unter einer physikalisch nahen Positionierung zu verstehen ist, dass eine Kurzbereichfunkeinrichtung oder ein Nähesensor in der medizinischen Vorrichtung (104, 326) oder dem ersten medizinischen Gerät (104, 328, 602) das Vorhandensein jeweils entsprechend des ersten medizinischen Geräts oder der medizinischen Vorrichtung erfasst, und
Kommunizieren der Konfigurationsinformationen an das erste medizinische Gerät in Antwort auf die Anfrage (606).

2. Verfahren nach Anspruch 1, das weiterhin das Beenden des Betriebs der medizinischen Vorrichtung (104, 326), nachdem die Konfigurationsinformationen kommuniziert wurden, aufweist.

3. Verfahren nach Anspruch 1, wobei das Starten des Betriebs, das Stoppen des Betriebs, das Überwachen des Fortschritts des Betriebs oder das Verwalten eines Alarms basierend auf dem Betrieb durch das zweite Gerät (102, 604) gesteuert werden.

4. Verfahren nach Anspruch 1, wobei das Kommunizieren das Übertragen der Konfigurationsinformationen zu dem ersten medizinischen Gerät (104, 328, 602) über das zweite Gerät (102, 604) aufweist.

5. Verfahren nach Anspruch 1, das weiterhin das Anpassen der Konfigurationsinformationen vor dem Kommunizieren mit dem ersten medizinischen Gerät (104, 328, 602) aufweist.

6. Verfahren nach Anspruch 5, wobei die Konfigurationsinformationen basierend auf dem Fortschritt des Betriebs der medizinischen Vorrichtung (104, 326) angepasst werden.

7. Verfahren nach Anspruch 1, das weiterhin aufweist:
Speichern der Konfigurationsinformationen in einem Speicher an der medizinischen Vorrichtung (104, 326), und
Abrufen der Konfigurationsinformationen aus dem Speicher in Antwort auf die Anfrage.

8. Verfahren nach Anspruch 1, wobei das erste medizinische Gerät eine Infusionspumpe (104, 328, 602) ist.

9. Eine medizinische Vorrichtung zum Replizieren von Konfigurationsinformationen in einer medizinischen Umgebung, **gekennzeichnet durch**:
einen Empfänger, der konfiguriert ist zum Empfangen einer Anfrage, dass Konfigurationsinformationen, die für das Konfigurieren der medizinischen Vorrichtung (104, 326) verwendet werden, in wenigstens einem ersten medizinischen Gerät (104, 328, 602), das die medizinische Vorrichtung ersetzen soll, repliziert werden,
wobei die Konfigurationsinformationen Patientenbehandlungsinformationen aufweisen,
eine Kurzbereichfunkeinrichtung zum Bewerkstelligen des Empfangens der Anfrage, wenn die medizinische Vorrichtung physikalisch nahe an dem ersten medizinischen Gerät positioniert ist, wobei unter einer physikalisch nahen Positionierung zu verstehen ist, dass eine Kurzbereichfunkeinrichtung oder ein Nähesensor in der medizinischen Vorrichtung (104, 326) oder dem ersten medizinischen Gerät (104, 328, 602) das Vorhandensein entsprechend jeweils des ersten medizinischen Geräts oder der medizinischen Vorrichtung erfasst,
ein Verarbeitungssystem, das konfiguriert ist zum Betreiben der medizinischen Vorrichtung basierend auf den Konfigurationsinformationen, wobei ein zweites Gerät konfiguriert ist zum Steuern des Betriebs, und
einen Sender, der konfiguriert ist zum Kommunizieren der Konfigurationsinformationen an das erste medizinische Gerät in Antwort auf das Empfangen der Anfrage durch den Empfänger.

10. Medizinische Vorrichtung nach Anspruch 9, wobei die medizinische Vorrichtung durch einen Patienten getragen wird oder in den Körper eines Patienten eingesetzt oder implantiert wird oder an dem Bett, der Trage oder der Kleidung des Patienten angebracht oder eingebettet wird.

11. Medizinische Vorrichtung nach Anspruch 9 oder 10, wobei die Konfigurationsinformationen wenigstens eines der Folgenden aufweisen:
Parameter für das Steuern der Vorrichtung,
den aktuellen Status des Betriebs der Vorrichtung,
in der Vorrichtung protokollierte Daten, oder
den Fortschritt einer durch die Vorrichtung verabreichten Behandlung.

12. Ein computerlesbares Medium mit darauf gespeicherten prozessorausführbaren Befehlen, die bei einer Ausführung durch einen Computer den Computer dazu veranlassen, das Verfahren gemäß einem der Ansprüche 1 bis 8 durchzuführen.

13. Ein erstes medizinisches Gerät zum Empfangen von replizierten Konfigurationsinformationen in einer medizinischen Umgebung, **gekennzeichnet durch**:
einen Sender, der konfiguriert ist zum Kommunizieren einer Anfrage, dass Konfigurationsinformationen, die für das Konfigurieren einer medizinischen Vorrichtung (104, 326) verwendet werden, in dem ersten medizinischen Gerät (104, 328, 602) repliziert werden,
wobei die Konfigurationsinformationen Patientenbehandlungsinformationen aufweisen,
Mittel zum Bestimmen, dass das erste medizinische Gerät physikalisch nahe an der medizinischen Vorrichtung positioniert ist, und zum Stellen der Anfrage in Antwort darauf, wobei unter einer physikalisch nahen Positionierung zu verstehen ist, dass eine Kurzbereichfunkeinrichtung oder ein Nähesensor in der medizinischen Vorrichtung oder dem ersten medizinischen Gerät das Vorhandensein jeweils der medizinischen Vorrichtung oder des ersten medizinischen Geräts erfasst,
einen Empfänger, der konfiguriert ist zum Empfangen der Konfigurationsinformationen, und
ein Verarbeitungssystem, das konfiguriert ist zum Betreiben des ersten medizinischen Geräts basierend auf den Konfigurationsinformationen unter der Steuerung eines zweiten Geräts.

## Revendications

1. Un procédé de reproduction d'informations de configuration au niveau d'un premier dispositif médical (104, 328, 602) dans un environnement de soins de santé, **caractérisé en ce qu'**il comprend :
la réception, au niveau d'un appareil médical (104, 326) actionné en fonction d'informations de configuration et commandé par un deuxième dispositif (102, 604), d'une demande relative aux informations de configuration utilisées de façon à configurer l'appareil médical (104, 326) à reproduire dans ledit premier dispositif médical (104, 328, 602),
où les informations de configuration comprennent des informations de traitement de patient,
où la demande est exprimée par la localisation physique du premier dispositif médical proche de l'appareil médical (104, 326), où localisé physiquement proche signifie qu'un dispositif radio à courte portée ou un capteur de proximité dans un élément parmi l'appareil médical (104, 326) et le premier dispositif médical (104, 328, 602) détecte la présence de l'autre élément parmi l'appareil médical et le premier dispositif médical, et
la communication des informations de configuration au premier dispositif médical, en réponse à la demande (606).

2. Le procédé selon la Revendication 1, comprenant en outre l'arrêt de l'actionnement de l'appareil médical (104, 326) une fois que les informations de configuration ont été communiquées.

3. Le procédé selon la Revendication 1, où au moins une opération parmi le démarrage de l'actionnement, l'arrêt de l'actionnement, la surveillance de la progression de l'actionnement ou la gestion d'une alarme en fonction de l'actionnement est commandée par le deuxième dispositif (102, 604).

4. Le procédé selon la Revendication 1, où la communication comprend le transfert des informations de configuration vers le premier dispositif médical (104, 328, 602) par l'intermédiaire du deuxième dispositif (102, 604).

5. Le procédé selon la Revendication 1, comprenant en outre l'ajustement des informations de configuration avant la communication avec le premier dispositif médical (104, 328, 602).

6. Le procédé selon la Revendication 5, où les informations de configuration sont ajustées en fonction de la progression de l'actionnement de l'appareil médical (104, 326).

7. Le procédé selon la Revendication 1, comprenant en outre :
la conservation en mémoire des informations de configuration dans une mémoire au niveau de l'appareil médical (104, 326), et
la récupération des informations de configuration à partir de la mémoire en réponse à la demande.

8. Le procédé selon la Revendication 1, où le premier dispositif médical est une pompe à perfusion (104, 328, 602).

9. Un appareil médical de reproduction d'informations de configuration dans un environnement de soins de santé, **caractérisé en ce qu'**il comprend :
un récepteur configuré de façon à recevoir une demande relative à des informations de configuration utilisées de façon à configurer l'appareil médical (104, 326) à reproduire dans au moins un premier dispositif médical (104, 328, 602) qui est destiné à remplacer l'appareil médical,
où les informations de configuration comprennent des informations de traitement de patient,
un moyen de radio à courte portée destiné à faciliter la réception de la demande si l'appareil médical est localisé physiquement proche du premier dispositif médical, où localisé physiquement proche signifie qu'un dispositif radio à courte portée ou un capteur de proximité dans un élément parmi l'appareil médical (104, 326) et le premier dispositif médical (104, 328, 602) détecte la présence de l'autre élément parmi l'appareil médical et le premier dispositif médical,
un système de traitement configuré de façon à actionner l'appareil médical en fonction des informations de configuration, où un deuxième dispositif est configuré de façon à commander l'actionnement, et
un émetteur configuré de façon à communiquer les informations de configuration au premier dispositif médical en réponse à la demande reçue par le récepteur.

10. L'appareil médical selon la Revendication 9, où l'appareil médical est porté sur soi par un patient, ou inséré ou implanté dans le corps d'un patient, ou fixé à ou imbriqué dans le lit, la civière roulante ou les vêtements d'un patient.

11. L'appareil médical selon la Revendication 9 ou 10, où les informations de configuration comprennent au moins des informations parmi :
des paramètres destinés à la commande de l'appareil,
un état d'actionnement actuel de l'appareil,
des données consignées dans l'appareil, ou
une progression d'un traitement administré par l'appareil.

12. Un support lisible par ordinateur possédant conservées en mémoire sur celui-ci des instructions exécutables par un processeur qui amènent un ordinateur à exécuter le procédé selon l'une quelconque des Revendications 1 à 8 lorsqu'elles sont exécutées par un ordinateur.

13. Un premier dispositif médical destiné à la réception d'informations de configuration reproduites dans un environnement de soins de santé, **caractérisé en ce qu'**il comprend :
un émetteur configuré de façon à communiquer une demande relative à des informations de configuration utilisées de façon à configurer un appareil médical (104, 326) à reproduire dans le premier dispositif médical (104, 328, 602),
où les informations de configuration comprennent des informations de traitement de patient,
un moyen de détermination que le premier dispositif médical soit localisé physiquement proche de l'appareil médical et d'expression de la demande en réponse à celle-ci, où localisé physiquement proche signifie qu'un dispositif radio à courte portée ou un capteur de proximité dans un élément parmi le premier dispositif médical et l'appareil médical détecte la présence de l'autre élément parmi le premier dispositif médical et l'appareil médical,
un récepteur configuré de façon à recevoir les informations de configuration, et
un système de traitement configuré de façon à actionner le premier dispositif médical en fonction des informations de configuration sous la commande d'un deuxième dispositif.
